# EUROPEAN PATENT SPECIFICATION

(11) **EP 3 547 995 B1**
(45) Date of publication and mention of the grant of the patent: **14.08.2024**
(21) Application number: 17879467.3
(22) Date of filing: 29.11.2017
(51) Int. Cl.: A61K 8/46, A61K 8/02, A61K 8/23, A61Q 5/08, A61K 8/25, A61K 8/41, A61K 8/44, A61Q 5/10

(54) **COMPOSITION FOR PROVIDING HIGH LIFT AND COLOR DEPOSIT**
ZUSAMMENSETZUNG ZUR BEREITSTELLUNG VON HOCHAUFTRIEB UND FARBABLAGERUNGEN
COMPOSITION PERMETTANT D'OBTENIR UN GRAND VOLUME ET UN DÉPÔT DE COULEUR

(30) Priority: 05.12.2016 US 201615369853
(43) Date of publication of application: 09.10.2019
(73) Proprietor: Henkel AG & Co. KGaA, 40589 Düsseldorf (DE)
(72) Inventor: AZIZOVA, Marina, New Canaan, Connecticut 06840 (US); ZHOU, Yanping, Blauvelt, NY 10913 (US); ABBAS, Razi, Bridgeport, CT 06604 (US)
(74) Representative: Petraz, Gilberto Luigi
(86) International application number: PCT/US2017/063579
(87) International publication number: WO 2018/106490

(56) References cited:
- WO-A1-03/084495
- WO-A2-01/72271
- WO-A2-2009/080670
- GB-A- 1 384 768
- US-A- 3 864 475
- US-A- 3 997 659
- US-A- 4 226 852
- US-A1- 2003 190 297
- US-A1- 2011 117 037
- US-A1- 2011 138 545
- US-A1- 2011 232 667
- US-A1- 2015 053 226
- US-A1- 2015 265 525
- US-B2- 7 905 926

## Description

### BACKGROUND OF THE INVENTION

### FIELD OF THE INVENTION

The invention relates to the field of systems and methods of lightening and/or coloring keratin-containing fibers, including mammal keratin-containing-containing fibers, and more particularly human hair. It is further particularly directed to systems and methods of lightening and/or coloring hair that include a lightening composition combined with an oxidizing agent for application to hair.

### DESCRIPTION OF RELATED ART

In the art of dyeing and/or lightening keratin-containing-containing fiber, particularly with respect to mammal, and more particularly with respect to human hair, various methods are used to lighten the color of hair and/or prepare the keratin-containing-containing fiber to receive a new color from a dyeing/coloring agent. In most traditional hair coloring compositions, alkalizing agents are used to attempt to increase depigmentation of hair, such as ammonium hydroxide, monoethanolamine, aminomethyl propanol with few variations in industry.

Metasilicates, also known as metasilicate salts, have been used in anhydrous hair bleaching compositions along with persulfate salts to lighten new hair growth. This material is also known for use in a wide variety of industrial and consumer applications as a buffer, corrosion inhibitor and as a pH stabilizer for bleaching. Metasilicate salts, such as sodium metasilicate, are not useful in water-based hair color compositions because they are not stable in aqueous systems in addition to their high irritation potential. Silicate salts are water soluble but only at high pH levels such as 10.9 -12. At lower pH, such salts are neutralized and precipitate out of solution as a silicic salt. Silicate salts further have other limitations, such as destabilizing emulsions by giving them an ionic shock and reacting with the amine groups in hair dyes, thus oxidizing them in the hair color compositions. All of these reasons make them disfavored choices for alkalizing. Metasilicate salts, while used in limited ways, as noted above, and while having a low toxicity, are known to have high skin sensitizing effects, and to cause irritation to the scalp and so are generally not employed in commercial hair coloring products for coloring and lightening (lifting) hair or in applications where the material comes in close and prolonged contact with the skin (scalp).

When used in hair coloring tubes to minimize tube corrosion, sodium metasilicate is used in small amounts typically of 0.01-0.1% which would not sufficiently contribute to hair lightening in the presence of peroxide.

US2011117037 A1 discloses a composition for bleaching and/or colouring the hair which enables reduced skin irritation as well as reduced hair damage, comprising alkanolamines, amino acids and associative polymers. The amino acid is preferably arginine. The composition is a lifting composition, ie. it achieves lightening as well as dyeing. The ammonia-free composition preferably comprises monoethanolamine with arginine, and preferably together with a metasilicate as additional alkalinising agent.

U.S. Patent Publication No. 2015/0265525 describes a hair coloring composition including an oxidizing composition for lifting or lightening hair color. If dye components are used, it can also color the hair while minimizing damage to the hair. The composition includes about 10-80% of at least one fatty substance; an acrylic polymer and about 0.5-15% of at least one salt which can be a variety of salts including an ammonium salt, a quaternary ammonium salt, a quaternary diammonium salt, an alkaline earth metal salt, a transition metal salt, an alkali metal salt, an agmatine salt and mixtures thereof. The composition includes at least one solvent. When the composition is mixed with an oxidizing agent or a composition thereof, the composition is said to improve hair color lightening or lifting effects. Amines may be used as in neutralizing agents, including alkanolamines and arginine.

U.S. Patent Publication No. 2014/0311517 A1 discloses methods and compositions for altering the color of hair. The hair is treated with a pre-alkalizing composition, and then treated with a color-altering composition that has at least one direct dye and at least one oxidizing agent. The system is described as addressing dryness, breakage and scalp irritation, particularly when multiple treatments are given. A method is described of applying a pre-alkalizing composition to the hair at pH of 8-14, optionally rinsing, and then applying a color-altering composition in a cosmetic carrier to the pre-alkalized hair. The color-altering composition includes at least one oxidizing agent (peroxide, persulfate, perborate, percarbonates, peracids, bromines and salts and mixtures thereof) and at least one direct dye with the pH at 1 to 7. The color-altering composition is left on to achieve color change and then the hair is rinsed. The color-altering composition may include a bleach and developer. The pre-alkalizing step may include a composition that lightens color. The composition may use of alkali metal salts, including sodium metasilicate, as a pH adjusting component for adjusting pH to no more than 7. The publication describes various lift-enhancing agents such as metal catalysts, ammonium salts, amino acids and urea. The amino acids may be, among others, arginine.

U.S. Patent No. 7,905,926 describes various alkali components for hair coloring compositions including among others alkali metasilicates, monoethanolamine, and diethanolamine. The components are added as "basifying" agents and may be used in amounts of 0.05-40% by weight based on the total weight of the color base composition and/or developer composition.

Great Britain Patent 1 384 768 teaches a hair bleaching composition which may use guanidine as an active ingredient. The patent notes that in the bleaching composition, ammonium hydroxide can irritate skin, but guanidine or a guanidine substitute such as arginine may be used to avoid irritation to skin and have less odor. The composition may also include water soluble alkali metal peroxides or other peroxides. Alkali metasilicates are suggested as thickeners.

U.S. Patent No. 3,861,868 describes an oxidation hair composition that includes peroxide and arginine in combination with an oxidation dye precursor in a diluents for coloring hair.

While such hair coloring and/or hair lifting compositions as noted above are known, there is a need in the art for a hair lightening (lifting) composition that may also be used with color in a single composition, that improves the level and efficiency of lift, provides ease of use, while also avoiding scalp irritation and hair damage of the recipient of the composition, and which may further aid in scalp soothing, without impacting the application of the color or an enhanced lift.

### BRIEF SUMMARY OF THE INVENTION

The present invention is set forth and characterized in the independent claims 1, 10 and 11, while the dependent claims describe other characteristics of the invention or variants to the main inventive idea. The disclosure includes systems for lightening and/or dyeing keratin-containing-containing fibers, lightening compositions for such systems and methods for applying such systems to keratin-containing-containing fibers. Disclosed is a system for dyeing and/or lightening keratin-containing-containing fibers comprising: (a) a first composition comprising (i) alkali components for lightening keratin-containing-containing fibers, wherein one of the alkali components comprises metasilicate, and the composition comprises about 0.1 to about 10 weight percent of the alkali components; and (ii) an arginine compound, wherein the arginine compound is present in an amount that would be effective to reduce irritation to a surface of a mammal having keratin-containing -containing fibers; and (b) an oxidizing agent composition comprising an oxidizing agent, wherein the system has a pH of at least about 9 to about 12.

In one embodiment, the first composition may be a liquid solution. Preferably, the metasilicate is sodium metasilicate. The first composition may comprise about 0.01 to about 3 percent by weight of the metasilicate.

The oxidizing agent may be hydrogen peroxide. The system preferably comprises about 0.1 to about 15% of the oxidizing agent. The system may further comprises at least one dye, which may be incorporated in the first composition.

The alkali components comprise ammonium hydroxide, ethanolamine, and optionally ammonium carbonate, aminomethyl propanol, monoisopropanol amine, and/or magnesium hydroxide.

The system may further comprise one or more conditioning agents that are stable in the first composition.

The system preferably comprises about 0.1 to about 10 percent by weight of the arginine compound. When the system is used on a scalp of a mammal having keratin-containing-containing fibers, the arginine compound is preferably present in an amount that aids in scalp soothing during a keratin-containing-containing fiber coloring process. The arginine compound may be selected from arginine salts, amino acids having an arginine component and/or polypeptides having an arginine component, and may in one embodiment comprise arginine carbonate, arginine phosphate and/or arginine chloride.

The disclosure further includes a lightening composition for making a system such as that noted above for dyeing and/or lightening keratin-containing-containing fibers. The lightening composition comprises: alkali components for lightening keratin-containing-containing fibers on a mammal, wherein one of the alkali components is metasilicate, and wherein the composition comprises about 0.1 to about 10 weight percent of the metasilicate.

In one embodiment of the lightening composition, the metasilicate is sodium metasilicate. The lightening composition also further comprises an arginine compound. When the lightening composition in such an embodiment is added to a system for dyeing and/or lightening keratin-containing-containing fiber, the arginine compound may comprise about 0.1 to about 10 percent by weight of the system. When the lightening composition is used in a system for dyeing and/or lightening keratin-containing-containing fiber and then used on a scalp of a mammal having keratin-containing-containing fiber, the arginine compound is preferably present in the system in an amount that aids in scalp soothing during a keratin-containing fiber coloring process. The arginine compound may be selected from arginine salts, amino acids having an arginine component and/or polypeptides having an arginine component, and in one embodiment the arginine compound comprises arginine carbonate, arginine phosphate and/or arginine chloride.

The lightening composition may further comprise a dye. In a further embodiment of the lightening composition, the metasilicate is sodium metasilicate and the composition includes the metasilicate in an amount of about 0.3 to about 3 percent. The alkali components comprise ammonium hydroxide, ethanolamine, and optionally ammonium carbonate, aminomethyl propanol, monoisopropanol amine, and/or magnesium hydroxide.

The lightening composition may further comprise one or more conditioning agents that are stable in the lightening composition.

The disclosure also includes a method for lightening keratin-containing fiber, comprising: (a) preparing a first composition comprising (i) the alkali components for lightening keratin-containing fiber, wherein the alkali components comprise metasilicate; and (ii) an arginine compound; (b) combining the first composition with an oxidizing agent to form a system having a pH of about 9 to about 12 and (c) applying the system to keratin-containing fiber to lighten the keratin-containing fiber.

In the method, in one embodiment, the metasilicate is sodium metasilicate and alkali components further comprise ammonium hydroxide, ethanolamine and optionally ammonium carbonate, aminomethyl propanol, monoisopropanol amine, and/or magnesium hydroxide

In an embodiment when the method is used on keratin-containing fibers of a mammal, the arginine compound is preferably present in the first composition in an amount effective to reduce irritation to a surface of a mammal having keratin-containing fibers. Further, in an embodiment when the method is used on keratin-containing fibers on a scalp of a mammal, the arginine compound may be present in the first composition in an amount that aids in scalp soothing during a keratin-containing fiber coloring process.

In an embodiment of an embodiment of the method, the arginine compound may be selected from arginine salts, amino acids having an arginine component and/or polypeptides having an arginine component. The arginine compound may also comprise arginine carbonate, arginine phosphate and/or arginine chloride.

In the method, the system may comprise about 0.1 to about 10 percent by weight of the arginine, and the first composition may comprise about 0.1 to about 10 percent by weight of the metasilicate.

In one embodiment of the method, step (a) of the method may further comprise the steps of preparing a liquid composition comprising the metasilicate; preparing a second oil phase composition; heating each of the liquid composition and the second oil phase composition; combining the liquid composition and the second oil phase composition to form a third composition; cooling the third composition; and adding a second alkali component from the alkali components, wherein the second alkali component is different from the metasilicate.

In this embodiment, the liquid composition and the second oil phase composition may be heated to about 75 °C to about 85 °C, although it is preferred that they are heated before combination. The step of adding the second alkali component further comprises adding a third alkali component from the alkali components, wherein the third alkali component is different than the second alkali component and different from the metasilicate. The second alkali component may be ethanolamine and the third alkali component may be ammonium hydroxide.

In one embodiment of the method, the metasilicate is sodium metasilicate, and preferably the metasilicate and the further alkali components provide a synergistic lightening of the keratin-containing fiber in comparison to either the metasilicate or the further alkali component each when added to the composition alone.

In an embodiment of the method, the CIELAB dE*ab value is preferably at least about 1 in comparison with a control.

In the method, the first composition may comprise one or more conditioning agents that are stable in the first composition.

In addition, in the method, step (c) may further comprise applying the system to a mammal having keratin-containing fibers and further applying conditioning agents, shampoos and/or conditioners to a mammal to promote softening and conditioning of a scalp of a mammal and/or keratin-containing fibers of a mammal.

### BRIEF DESCRIPTION OF THE SEVERAL VIEWS OF THE DRAWING

The foregoing summary, as well as the following detailed description of preferred embodiments of the invention, will be better understood when read in conjunction with the appended drawings. For the purpose of illustrating the invention, there is shown in the drawings embodiments which are presently preferred. It should be understood, however, that the invention is not limited to the precise arrangements and instrumentalities shown. In the drawings:

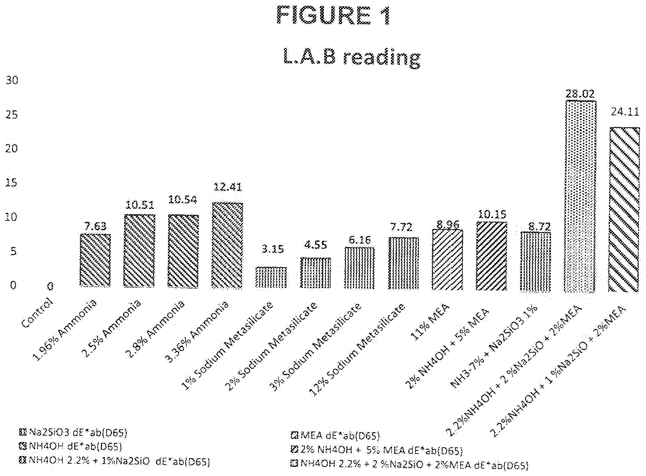

Fig. 1 is a chart presenting data from Example 2 and Table 7.

### DETAILED DESCRIPTION OF THE INVENTION

This disclosure provides for a hair lightening and/or color system, methods for hair lightening and/or coloring and lightening compositions for use in such systems and methods. The systems and methods herein provide the benefit of and enable use of a metasilicate to achieve high levels of lightening and depigmentation of keratin-containing fibers in ammoniated and ammonia-free lightening and/or coloring applications, while offering a solution to relieve scalp irritation, prevent damage to keratin-containing fiber and/or to soothe the scalp during application for safe use of a metasilicate. The method provides a path for incorporating a metasilicate into a system for lightening and/or coloring keratin-containing fibers.

The invention herein also provides a system, method and lightening composition therefor that can incorporate sodium metasilicate in a stable, effective and safe hair coloring system. The applicants herein in evaluating various compositions to create a system successfully incorporating metasilicate also surprisingly found that various combinations of alkalizing agents with a metasilicate provide synergistic results wherein the combination acts to buffer and stabilize the peroxide -OH radicals in the systems herein making them available for a longer period of time to effectively lighten the natural pigment of the keratin-containing fibers.

In the present invention, also it is possible to employ a water-soluble alkaline metasilicate to provide additional lift in an ammonia-free system based on ethanolamine. Such a system mixed with hydrogen peroxide in a pH range of about 9.5 to about 12 resembles a traditional hair color system, with no odor, and that also provides significantly higher lift than an ethanolamine-alkali system alone.

In referring to a "metasilicate," for use in the invention herein, applicant intends to incorporate alkaline silicates, preferably in salt form, that function within the nature and scope of the proposed compositions and methods for lightening and coloring hair. The term is not meant to be limiting. A preferred metasilicate for use herein is sodium metasilicate, and sodium metasilicate may be referred to generally herein for exemplary purposes, but it should be understood that other alkaline metasilicates that demonstrate the same or similar properties may be used within the scope of the invention. Examples of such silicates that may be used as the metasilicate in the present invention include aluminum silicate, calcium silicate, magnesium aluminum silicate, magnesium silicate, magnesium trisilicate, sodium magnesium silicate, zirconium silicate, attapulgite, bentonite, Fuller's earth, hectorite, kaolin, lithium magnesium silicate, lithium magnesium sodium silicate, montmorillonite, pyrophyllite and zeolite, the latter of which are clay-like ingredients that are based on silicates.

The alkaline metasilicate(s) herein may also be added to an ammonia-based hair coloring system that mixes an oxidizing agent such as hydrogen peroxide in a pH range of about 9.5 to about 12 to create a system like a traditional hair coloring system, but that provides additional lightening without adding more ammonia and thus minimizing the odor in the final product.

In the systems herein, the increase of alkali and use of metasilicate(s) such as sodium metasilicate, can lead to scalp sensitivity due to a high level of alkalinity. The systems and methods herein preferably include an arginine compound to offer relief from scalp irritation and/or to soothe the scalp and protect it for a consumer-friendly use.

Shown below is a typical molecule of a sodium metasilicate salt:

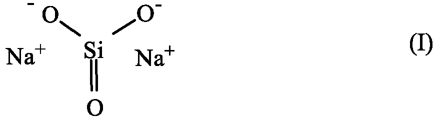

Also shown below is a typical, exemplary arginine amino acid structure:

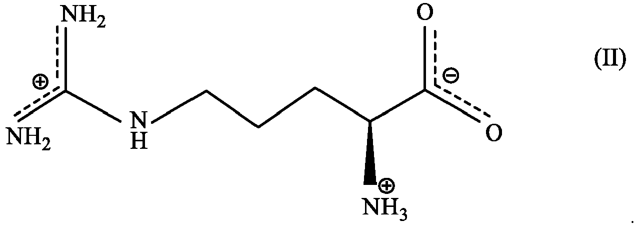

An arginine compound as that term is used herein is meant to apply to arginine; compounds of arginine, including arginine salts; amino acids and/or polypeptides which have arginine components therein; derivatives thereof (such as modified or functionalized arginine compounds that are otherwise still compatible with or add additional benefits to those discussed herein). Some of such compounds are generally described in U.S. Patent No. 3,861869. Preferred arginine compounds include 1-arginine, arginine carbonate, arginine phosphate and/or arginine chloride.

It is within the scope of the disclosure herein to also optionally use one or more acceptable arginine substitutes either instead of or in addition to the use of the arginine compounds noted herein (which can encompass amino acids and similar compounds that can perform the same function as the arginine materials described herein). For example, but not to be limiting, applicant herein refers to materials described in Great Britain Patent No. 1 384 768 for listing possible arginine substitutes

Arginine metabolism plays an important role in aspects of inflammation and wound healing. The true mechanism of arginine in wound healing is not believed to be identified, however, it is demonstrated to help in healing wounds and minimizing inflammation. As used in the present invention's systems and methods, the arginine compound is important to increasing scalp comfort by buffering the so as to minimize irritation from the highly alkaline use of materials such as ammonium hydroxide, ethanolamine and sodium metasilicate and/or its salt.

In the systems herein for coloring and/or lightening keratin-containing fibers, the use of the words "dyeing" and "coloring" are used to refer to applying a new color and/or changing a color of a keratin-containing fiber. The terms "lift" or "lifting" relates to the lightening of the hair. Use of "lift" and "lightening" herein mean the same thing and the terms may be used interchangeably.

The reference herein to a "keratin-containing fiber" is intended to refer to keratin or any keratinic fiber, including mammal hair, for humans and mammals, including, but limited to hair, fur, sheep wool, feathers, and in particular human hair. While the invention was developed for keratin-containing fiber, and for coloring human hair, this should not exclude use of the invention for other potential coloring and/or lifting end applications.

In the systems, herein, the system includes a first composition that includes alkali components for lightening keratin-containing fibers. One of the alkali components herein includes a metasilicate, such as alkaline metasilicates, including sodium metasilicate as noted above. In forming this composition for use in the system, the composition is used preferably as a lightening composition. The metasilicate along with the other alkali components as described below are preferably present in the lightening composition in a total amount of about 0.1 to about 10 weight percent. The metasilicate(s) is/are preferably present in the lightening composition in an amount of about 0.01 to about 3 percent by weight.

The composition for lightening is preferably a liquid lotion or cream composition, but need not be and powder compositions are within the general scope of the invention. As a liquid composition, it is preferred that the solvent base is an aqueous in nature. However, other systems could be used having a different solvent such as an alcohol or organic solution base, e.g., silicone, glycerine, or glycols such as propylene glycol, provided the solvent selected does not impact one or more of the beneficial properties achieved by the systems and/or the components thereof as described herein.

The alkali component herein has alkali components in combination, wherein one of the alkali components is an alkaline metasilicate(s) such as one of the metasilicate examples listed above. Other alkali component(s) may be various agents known in the art, as claimed, including ammonia; alkanolamines, such as monoethanolamine (MEA), methanolamine, monoisopropanolamine and aminomethyl propanol; other alkali metal compounds or alkali metal silicic compounds and their salts, and calcium hydroxide, sodium hydroxide, magnesium hydroxide, lithium hydroxide, barium hydroxide, potassium hydroxide, and similar compounds and their salts, and other silicates such as the metasilicates noted above with respect to the metasilicate component; as well as combinations, salts, or derivatives thereof. Also useful are ammonium carbonate and/or ammonium bicarbonate, and some amino acids in some instances. The alkali component for use with the metasilicate component includes monoethanolamine (MEA) and ammonium hydroxide, and optionally ammonium carbonate, aminomethyl propanol, monoisopropanol amine, and/or magnesium hydroxide. Such combinations contribute to a synergistic lightening effect.

An arginine compound, as defined above, is also provided to the composition. The amount of arginine may vary, but it is preferred that it be present in an amount that is effective to reduce irritation to a surface of a mammal having keratin-containing fibers. In other words, there should be sufficient arginine compound to counteract irritation and further, it is preferred that the arginine compound is present also in an amount in the system that aids in scalp soothing during a keratin-containing fiber coloring process.

In preferred embodiments, the arginine compound is added so that it will make up about 0.1 to about 10 percent by weight, and preferably 0.1 to about 0.5 percent by weight, of the combined system that incorporates the lightening composition herein. The arginine compound is more preferably present in the system in an amount that aids in scalp soothing during a keratin-containing fiber coloring process, including but not limited to amounts of about 2 to about 3 percent by weight percent.

As noted above, in addition to the above-lightening composition, the system includes a further oxidizing agent composition that includes primarily an oxidizing agent. When the oxidizing agent composition and the lightening composition are combined to prepare the system, it is preferred that the system have a pH of at least about 9 to about 12.

The preferred oxidizing agent is hydrogen peroxide, however, other known oxidizing agents may be used such as air/oxygen, organic and inorganic peroxides, including alkali salts of perborates, percarbonates, perphosphates, persulfates and the like, as well as salts and hydrates thereof. In the present system, however, hydrogen peroxide is most preferred.

In the system, the oxidizing agent, such as hydrogen peroxide, should be added to the oxidizing agent composition so that it will make up about 0.1 to about 15 weight percent, and preferably about 3 to about 12 percent by weight.

Other optional components for the system, including the use of at least one dye for coloring hair as well as one or more conditioning agents that are stable in the first composition. Some of the optional components may be added to the oxidizing composition, but most are preferably added to the lightening composition, particularly the coloring additives such as dyes and dye precursors to be activated when combined and applied to hair.

In the lightening composition, if a dye and/or a dye precursor is incorporated, a carrier for the dye may be used, in addition to water, typically such a carrier is a solvent that is miscible in water. Such components are known in the art, and include alkanols and/or alkoxy alkanes, such as ethers, including short chain organic alcohols, glycols and ethers which are suitable for use in and compatible and stable in a hair lightening and/or coloring composition. Dyes and/or dye or dye precursors are optional but preferred in the composition. Any dye(s) and/or dye precursor(s) that are known or to be developed which are suitable for use in a one-step lightening and/or coloring formulation having peroxide as described herein may be used. Commonly used dyes are amine and phenol based compounds, such as p-phenylenediamine, resorcinol, p-aminophenol, p-toluene-diamine, azo dyes and red dyes. Examplary dyes include, but are not limited to p-phenylenediamine, p-aminophenol, N,N-bis-(2-hydroxyethyl)-p-phenylenediamine sulfate, 4-amino-m-cresol (3-methyl-4-aminophenol), 6-amino-m-cresol, 1-hydroxyethyl 4,5-diamino pyrazole sulfate, tetraaminopyrimidine sulfate, toluene-2,5-diamine sulfate, resorcinol, 4-chlororesorcinol, m-aminophenol, 2-methylresorcinol, 4-amino-2-hydroxytoluene(p-amino-o-cresol), 2-methyl-5-hydroxyethylaminophenol, 2-amino-3-hydroxypyridine, 1-naphthol, 5-amino-6-chloro-o-cresol, 2,4-diaminophenoxyethanol HCl, 2-amino-4-hydroxyethylaminoanisole sulfate, 2,6-dihydroxyethylaminotoluene, 1,2,4-trihydroxybenzene, 1-phenyl-3-methyl-5-pyrazolone, 2,6-diaminopyridine, direct dyes, 3-nitro-p-hydroxyethylaminophenol, 2-amino-6-chloro-4-nitrophenol, 4-nitro-o-phenylenediamine, HC Blue 2, HC Blue No. 16, HC Yellow 4, HC Red 3, HC Yellow #2, 2-hydroxyethylamino-5-nitroanisole, HC Yellow 7, N,N'-bis(2-hydroxyethyl)-2-nitro-p-phenylenediamine, 2-nitro-p-phenylenediamine, 2-hydroxyethylamino-5-nitroanisole, HC Yellow 7, N,N'-bis(2-hydroxyethyl)-2-nitro-p-phenylenediamine, and 2-nitro-*p-*phenylenediamine.

Other optional components may include cationic surfactants (such as amines), nonionic surfactants, such as fatty acids or alcohols, polyethylene glycols, anionic surfactants and similar materials which are known in the art for this purpose and suitable for use in, compatible with and stable within hair coloring compositions.

Further optional components include dispersants known or to be developed in the art, thickeners, rheological agents, chelating agents, antioxidants, conditioning agents stable within the composition and system, viscosity modifiers, emulsifiers, fragrance components, preservatives, and similar materials.

The lightening composition in the system may be used independently in this system or in other systems, and can be provided as part of a system for combination and application to hair in a one-step lightening and coloring product or as a separate component product. The lightening composition and oxidizing agent composition may be sold as independent products together as a system in the form of a kit including user instructions setting forth the steps for combining and applying the compositions and combined system to hair following the method as described below.

In the method for lightening keratin-containing fiber herein, the method includes first preparing a first composition that includes said alkali components for lightening keratin-containing fiber. The first composition prepared in the method is preferably the lightening composition noted above. The alkali components include the metasilicate as noted above and further the other alkali components as noted above. The first composition also incorporates one or more arginine compound(s) such as those noted above for use alone or in combination in the lightening composition herein.

The first composition is combined with an oxidizing agent (which can be added in various forms such as in an oxidizing agent composition as noted above) to form a system having a pH of about 9 to about 12. The combined system is then applied to keratin-containing fiber, such as human hair, to lighten (lift) the hair. The various components are as described above and are preferably added in the amounts noted above.

When forming the first composition which is preferably the lightening composition, in one embodiment herein, a liquid composition is preferably prepared that includes the metasilicate, preferably sodium metasilicate although other alkaline metasilicates may be used as noted above. A second, oil phase composition is also prepared. Each of the compositions is heated, preferably before combination of the compositions. The liquid composition and the second oil phase composition may be heated to about 75 °C to about 85 °C and then combined to form a third (intermediate) composition which is preferably cooled. The second and third or other additional alkali component(s) are then added, each of which should be different from the metasilicate in the initial liquid composition and different from each other. Other steps may of course be used, and this embodiment is not intended to be limited.

The additional alkali component(s), include ammonium hydroxide as a second alkali component and monoethanolamine as the third alkali component. The metasilicate and the second alkali component together provide a preferred synergistic lightening of the keratin-containing fiber in comparison to either the metasilicate or the second alkali component each when added to the composition alone.

As demonstrated below in the Examples, the resulting lightening composition for use in the systems and methods herein has a CIELAB dE*ab value which is at least about 1 when comparing it with a control.

The method may also include, when applying the system to a mammal having keratin-containing fibers, either with the application or before or after, further applying conditioning agents, shampoos and/or conditioners to the hair and scalp to promote softening and conditioning of a scalp and/or hair.

The invention will now be described in accordance with the following, nonlimiting examples.

### EXAMPLE 1

The following represent various example hair color bases formed according to the invention. Table 1 includes an example of a hair color base A with sodium metasilciate without dyes.

**Table 1**

| Ingredient INCI Names | Functionality | Weight % |
|---|---|---|
| Batch: Heat it up to 75C to 80C | | |
| Water | Solvent | q.s to 100 |
| Propylene Glycol | Humectant | 3.0% |
| Sodium Sulfite | Antioxidant | 0.4% |
| Sodium Isoascorbate | Antioxidant | 0.4% |
| EDTA | Chelating Agent | 0.14% |
| Sodium Metasilicate | Alkalizer | 2.0% |

| Oil Phase: Heat it up to 75C to 80C | | |
|---|---|---|
| Cetyl Alcohol | Opacifying Agent | 2.2 |
| Stearic Acid | Surfactant-Cleansing Agent | 1.8 |
| Glyceryl Stearate | Surfactant-Emulsifying Agent | 0.28 |
| Steareth-21 | Surfactant-Cleansing Agent | 2.5 |
| | | |
| Glycol distearate | Opacifying Agent | 0.28 |
| Steramindopropyl dimethyl amine | Hair conditioning Agent | 1.35 |
| Mineral Oil | Hair Conditioning Agent | 1 |
| Isostearic acid | Binder | 0.7 |
| Oleyl Alcohol | Emollient | 0.1 |
| Polysorbate 80 | Surfactant-Emulsifying Agent | 0.24 |
| Once the Batch and Oil Phase reaches to 75C -80C mix them together. After the batch is mixed, start cooling the batch and once it reaches 35C add fragrance and the alkalizers. | | |
| Fragrance | Perfume | 0.6 |
| Ethanolamine | Alkalizer | 2 |
| Ammonium hydroxide (28% solution) | Alkalizer | 8 |

Further examples below are formed using the same procedure as noted above in Table 1. Table 2 shows an example of a hair color base including sodium metasilicate and arginine without dyes.

**Table 2**

| Ingredient INCI Names | WEIGHT % |
|---|---|
| Water | q.s to 100 |
| Propylene Glycol | 3.0% |
| Sodium Sulfite | 0.4% |
| Sodium Isoascorbate | 0.4% |
| EDTA | 0.14% |
| L-Arginine | 2.0% |
| Sodium Metasilicate | 2.0% |
| Cetyl Alcohol | 2.2 |
| Stearic Acid | 1.8 |
| Glyceryl Stearate | 0.28 |
| Steareth-21 | 2.5 |
| Glycol distearate | 0.28 |
| Steramindopropyl dimethyl amine | 1.35 |
| Mineral Oil | 1 |
| Isostearic acid | 0.7 |
| Oleyl Alcohol | 0.1 |
| Polysorbate 80 | 0.24 |
| Fragrance | 0.6 |
| Ethanolamine | 8 |

Table 3 includes an example of a hair color base (medium red) with sodium metasiliciate and arginine.

**Table 3**

| Ingredient INCI Names | WEIGHT % |
|---|---|
| Water | q.s to 100 |
| Propylene Glycol | 3.0% |
| Sodium Sulfite | 0.4% |
| EDTA | 0.14% |
| L-Arginine | 1.0% |
| 1-Naphthol | 0.46 |
| m-Aminophenol | 0.528 |
| 1-Hydroxyethyl 4,5-Diamino Pyrazole Sulfate | 1.62 |
| Sodium Metasilicate | 2.0% |
| Cetyl Alcohol | 2.2 |
| Stearic Acid | 1.8 |
| Glyceryl Stearate | 0.28 |
| Steareth-21 | 2.5 |
| Glycol distearate | 0.28 |
| Steramindopropyl dimethyl amine | 1.35 |
| Mineral Oil | 1 |
| Isostearic acid | 0.7 |
| Fragrance | 0.6 |
| Ethanolamine | 2 |
| Ammonium hydroxide (28% solution) | 8 |

Table 4 includes an example of a hair color shade (medium ash) with sodium metasilicate and arginine.

**Table 4**

| Ingredient INCI Names | WEIGHT % |
|---|---|
| Water | q.s to 100 |
| Propylene Glycol | 3.0% |
| Sodium Sulfite | 0.4% |
| Sodium Isoascorbate | 0.4% |
| EDTA | 0.14% |
| L-Arginine | 2.0% |
| p-Phenylenediamine | 0.33 |
| Hydroxyethyl-3,4-Methylenedioxyaniline HCl | 0.5 |
| 2,4-Diaminophenoxyethanol HCl | 0.065 |
| Resorcinol | 0.13 |
| N,N-Bis(2-Hydroxyethyl)-p-Phenylenediamine Sulfate | 0.032 |
| Sodium Metasilicate | 3.0% |
| Cetyl Alcohol | 2.2 |
| Stearic Acid | 1.8 |
| Glyceryl Stearate | 0.28 |
| Steareth-21 | 2.5 |
| Glycol distearate | 0.28 |
| Steramindopropyl dimethyl amine | 1.35 |
| Mineral Oil | 1 |
| Isostearic acid | 0.7 |
| Oleyl Alcohol | 0.1 |
| Polysorbate 80 | 0.24 |
| Fragrance | 0.6 |
| Ethanolamine | 2 |
| Ammonium hydroxide (28% solution) | 8 |

### EXAMPLE 2

The examples above show hair lightening and hair coloring compositions. They were further prepared using varying amounts of mono-isopropanol amine (MIPA), monoethanolamine (MEA), aminomethyl proponol (AMP), ammonium carbonate, ammonium bicarbonate, ammonium hydroxide, magnesium hydroxide and sodium metasilicate individually as shown below in Tables 5 and 6, and were evaluated to observe which alkalizer would provide the highest levels of lightening of the dark hair. Furthermore, from Tables 5 and 6 it was concluded that ammonium hydroxide is the strongest alkali that provided the highest amount of lift on the darker hair. Table 5 represents a visual evaluation of the various alkalizers' lightening power.

**Table 5**

| Alkalizers at various levels | As is pH | RTA pH | Lightening power | Performance rating* |
|---|---|---|---|---|
| Ammonium Hydroxide (NH₄OH) 3.4% | 11.04 | 10.5 | Strongest | 5 |
| Ethanolamine (MEA) 11% | 11.09 | 10.57 | Strong | 4 |
| Sodium Metasilicate (Na₂SiO₃) 12% | 12.6 | 11.32 | Strong | 3.5 |
| Ammonium Carbonate (NH₄CO₃) 10% | 9.11 | 8.95 | Weak | 3 |
| Aminomethyl propanol( AMP) 11% | 11 | 10.23 | Weak | 2.5 |
| Mono Isopropanol amine (MIPA) 10% | 11 | 10.13 | Weaker | 2 |
| Magnesium hydroxide (MgOH₂) 10% | 9.87 | 8.95 | Weakest | 1 |

| | | | | |
|---|---|---|---|---|
| *Performance rating scale: strongest 5 (most amount of lightening/lift) and weakest 1 (least amount) | | | | |

Table 6 below shows the Minolta L.A.B. evaluation of the various alkalizers' lightening power.

**Table 6 Minolta L.A.B evaluation of alkalizers' lightening power**

| Data Name | L*(D65) | a*(D65) | b*(D65) | dL*(D65) | da*(D65) | db*(D65) | dE*ab(D65)* |
|---|---|---|---|---|---|---|---|
| Black Control Tress | 20.38 | 1.53 | 1.59 | ------ | ------ | ------ | ------ |
| NH₄OH 3.4% | 25.69 | 7.86 | 10.84 | 5.31 | 6.33 | 9.25 | 12.41 |
| MEA 11% | 23.03 | 6.52 | 8.01 | 2.65 | 4.99 | 6.43 | 8.56 |
| NH₄OH 2% | 23.25 | 5.87 | 7.17 | 2.87 | 4.34 | 5.58 | 7.63 |
| Na₂SiO₃ 12% | 23.23 | 5.97 | 7.22 | 2.85 | 4.44 | 5.63 | 7.72 |
| NH₄CO₃ 10% | 22.52 | 5.68 | 6.94 | 2.14 | 4.15 | 5.36 | 7.1 |
| AMP 11% | 21.26 | 4.8 | 5.34 | 0.88 | 3.27 | 3.75 | 5.05 |
| MIPA 10% | 20.55 | 4.68 | 5.09 | 0.17 | 3.15 | 3.5 | 4.71 |
| MgOH₂ 10% | 19.55 | 2.3 | 2.45 | -0.83 | 0.77 | 0.86 | 1.42 |

| | | | | | | | |
|---|---|---|---|---|---|---|---|
| *Higher Reading Delta E (dE*ab) corresponds to higher lightening of black hair | | | | | | | |

As ammonium hydroxide provided the highest lift, it could be used by itself, however at higher levels, the ammonia odor becomes an issue for consumers. Thus, to maximize the lightening effect without increasing ammonia to the higher levels, the inventors herein combined it with the second and third best alkalizers from Table 5, i.e., ethanolamine and sodium metasilicate. Surprisingly, when added to other alkalizers, metasilicate salts can provide much higher lightening due its ability to stabilize OH-radicals and enhance lightening of the hair's melanin.

The following experiments were done with various combinations of ammonium hydroxide, MEA, metasilicate, etc. to find the best way to lighten hair. Higher Delta E (dE*ab) readings correspond to higher lightening of hair from the control. In all examples, the ΔE values were measured using a L.A.B. reading using the color measurement method of CIELAB which is used to mathematically compute and compare the color of hair tresses. The method was developed by CIE using L*, a* and b* values. L* represents the difference between light (where L* = 100) and dark (where L* is 0); a represents the difference between green (-a*) and red (+a*); and b* represents the difference between yellow (+b*) and blue (-b*). This system then maps true color to a place on a three dimensional graph wherein the variables are calculated by the changes in their values and those changes are used to compute the ΔE value which is a change in color. Such a system is commercially known and used in the art.

Table 7 below and Figure 1 include L.A.B. readings for base prototypes and shows the synergistic effect provided by Sodium Metasilicate as an additional alkalizer in ammonia and MEA-based systems.

**Table 7**

| Data Name | L*(D65) | a*(D65) | b*(D65) | dL*(D65) | da*(D65) | db*(D65) | dE*ab(D65) |
|---|---|---|---|---|---|---|---|
| Baseline-Black Tress | 20.38 | 1.53 | 1.59 | ------ | ------ | ------ | ------ |
| NH4OH -2.2 % + MEA 2% + Na2SiO3 2% | 37.13 | 10.19 | 22.31 | 16.75 | 8.66 | 20.72 | 28.02 |
| NH4OH -2.2 % + MEA 2% + Na2SiO3 1% | 33.44 | 10.3 | 19.86 | 13.06 | 8.77 | 18.27 | 24.11 |
| NH4OH 3.36% | 25.69 | 7.86 | 10.84 | 5.31 | 6.33 | 9.25 | 12.41 |
| NH4OH 2.5% | 25.33 | 6.98 | 9.08 | 4.95 | 5.45 | 7.5 | 10.51 |
| NH4OH 1.96% | 23.25 | 5.87 | 7.17 | 2.87 | 4.34 | 5.58 | 7.63 |
| MEA 11% | 23.03 | 6.52 | 8.01 | 2.65 | 4.99 | 6.43 | 8.56 |
| NH4OH 2.2% + MEA 5% | 24.34 | 7.06 | 9.12 | 3.96 | 5.53 | 7.53 | 10.15 |
| Na2SiO3 12% | 22.53 | 5.78 | 7.04 | 2.15 | 4.25 | 5.46 | 7.24 |
| Na2SiO3 1% | 27.05 | 5.47 | 9.16 | 2.09 | 1.05 | 2.12 | 3.15 |
| Na2SiO3 2% | 27.98 | 5.99 | 10.06 | 3.01 | 1.58 | 3.02 | 4.55 |
| Na2SiO3 3% | 29.44 | 6.32 | 10.83 | 4.47 | 1.91 | 3.79 | 6.16 |
| NH4OH 2% + Na2SiO3 1% | 23.83 | 6.41 | 7.94 | 3.45 | 4.88 | 6.35 | 8.72 |

The commonly used, highest amount of ammonium hydroxide (in high-lift shades with 3.4% ammonia) provided a lightening level corresponding to L.A.B. Reading 12.41. The optimized system with ammonium hydroxide at 2.2%, MEA at 2% and 1% sodium metasilicate allowed the inventors to achieve an L.A.B. Reading of 24.11. This would not be possible without the synergistic effect of sodium metasilicate. Furthermore, when the amount of sodium metasilicate was increased to 2%, the inventors were able to achieve a L.A.B. reading of 28.02.

As shown graphically in Figure 1, the addition of sodium metasilicate to ammonium hydroxide and MEA consistently showed synergistic lift over the same component when used alone. It finally became possible to match and exceed the lift of ammonium hydroxide, which is known to be the most effective alkalizing and lifting agent.

## Claims

1. A system for dyeing and/or lightening keratin-containing fibers comprising:
(a) a first composition comprising
(i) alkali components for lightening keratin-containing fibers, wherein the alkali components are present in an amount of about 0.1 to about 10 weight percent of the first composition, wherein the alkali components comprise a metasilicate, ammonium hydroxide, and monoethanolamine, and wherein the first composition comprises about 0.01 to about 3 weight percent of the metasilicate; and
(ii) an arginine compound, wherein the arginine compound is present in the system in an amount of about 0.1 to about 10 weight percent for reducing irritation to a surface of a mammal having keratin-containing fibers; and
(b) an oxidizing agent composition comprising an oxidizing agent, wherein the system has a pH of at least about 9 to about 12, wherein the metasilicate, ammonium hydroxide and monoethanolamine provide a synergistic lightening of the keratin-containing fiber in comparison to each of the metasilicate, ammonium hydroxide, or monoethanolamine when added to the composition alone.

2. The system according to claim 1, wherein the metasilicate is sodium metasilicate.

3. The system according to claim 1 or 2 , wherein the first composition is a liquid solution.

4. The system according to any of the previous claims, wherein the oxidizing agent is hydrogen peroxide.

5. The system according to any of the previous claims, wherein the system comprises about 0.1 to about 15% of the oxidizing agent.

6. The system according to any of the previous claims, wherein the system comprises at least one dye.

7. The system according to any of the previous claims, wherein the alkali components further comprise one or more of ammonium carbonate, aminomethyl propanol, monoisopropanol amine, and magnesium hydroxide.

8. The system according to any of the previous claims, further comprising one or more conditioning agents that are stable in the first composition.

9. The system according to any of the above claims, wherein the arginine compound is selected from arginine salts, amino acids having an arginine component and/or polypeptides having an arginine component, and preferably arginine carbonate, arginine phosphate and/or arginine chloride.

10. A lightening composition for making a system for dyeing and/or lightening keratin-containing fibers, the lightening composition comprising:
alkali components for lightening keratin-containing fibers on a mammal, wherein the alkali components comprise a metasilicate, ammonium hydroxide, and monoethanolamine, and wherein the composition comprises about 0.1 to about 3 weight percent of the metasilicate, wherein the metasilicate, ammonium hydroxide, and monoethanolamine provide a synergistic lightening of the keratin-containing fiber in comparison to each of the metasilicate, ammonium hydroxide or the monoethanolamine when added to the composition alone.

11. A non-therapeutic method for lightening keratin-containing fiber, comprising:
(a) preparing a first composition comprising (i) alkali components for lightening keratin-containing fiber, wherein the alkali components comprise metasilicate, ammonium hydroxide, and monoethanolamine; and (ii) an arginine compound;
(b) combining the first composition with an oxidizing agent to form a system having a pH of about 9 to about 12 and
(c) applying the system to keratin-containing fiber to lighten the keratin-containing fiber, wherein the metasilicate, ammonium hydroxide, and monoethanolamine provide a synergistic lightening of the keratin-containing fiber in comparison to each of the metasilicate, ammonium hydroxide or monoethanolamine when added to the composition alone.

12. The method according to claim 11, wherein the metasilicate is sodium metasilicate and the at least two alkali components further comprise ammonium carbonate, aminomethyl propanol, monoisopropanol amine, and magnesium hydroxide.

13. The method according to claim 11, wherein step (a) further comprises
preparing a liquid composition comprising the metasilicate;
preparing a second oil phase composition;
heating each of the liquid composition and the second oil phase composition;
combining the liquid composition and the second oil phase composition to form a third composition;
cooling the third composition; and
adding the ammonium hydroxide and monoethanolamine.

14. The method according to claim 13, wherein the liquid composition and the second oil phase composition are each heated to about 75 °C to about 85 °C prior to combining the liquid composition and the second oil phase composition.

15. The method according to claim 13 or 14, wherein the step of adding the ammonium hydroxide and the monoethanolamine further comprises adding a further alkali component that is different than the metasilicate, the ammonium hydroxide and the monoethanolamine.

16. The method according to claim 15, wherein the CIELAB dE*ab value is at least about 1 in comparison with a control.

17. The method according to any of claims 11 to 16, wherein step (c) further comprises applying the system to a mammal having keratin-containing fibers and further applying conditioning agents, shampoos and/or conditioners to a mammal to promote softening and conditioning of a scalp of a mammal and/or keratin-containing fibers of a mammal.

## Patentansprüche

1. System zum Färben und/oder Aufhellen von keratinhaltigen Fasern, welches aufweist:
(a) eine erste Zusammensetzung, welche aufweist:
(i) Alkalikomponenten zum Aufhellen keratinhaltiger Fasern, wobei die Alkalikomponenten in einer Menge von etwa 0,1 bis etwa 10 Gewichtsprozent der ersten Zusammensetzung vorhanden sind, wobei die Alkalikomponenten Metasilicat, Ammoniumhydroxid und Monoethanolamin aufweisen, und wobei die erste Zusammensetzung etwa 0,01 bis etwa 3 Gewichtsprozent des Metasilicats aufweist, und
(ii) eine Argininverbindung, wobei die Argininverbindung in dem System in einer Menge von etwa 0,1 bis etwa 10 Gewichtsprozent vorhanden ist zum Verringern von Irritation an einer Oberfläche eines Säugetiers, welche keratinhaltige Fasern hat, und
(b) eine Oxidationsmittelzusammensetzung, welche ein Oxidationsmittel aufweist, wobei das System einen pH-Wert von mindestens etwa 9 bis etwa 12 hat, wobei das Metasilicat, das Ammoniumhydroxid und das Monoethanolamin eine synergistische Aufhellung der keratinhaltigen Faser im Vergleich zu jedem des Metasilicats, Ammoniumhydroxids oder Monoethanolamins, wenn sie der Zusammensetzung allein zugesetzt werden, bewirken.

2. System nach Anspruch 1, wobei das Metasilicat Natriummetasilicat ist.

3. System nach Anspruch 1 oder 2, wobei die erste Zusammensetzung eine flüssige Lösung ist.

4. System nach irgendeinem der vorhergehenden Ansprüche, wobei das Oxidationsmittel Wasserstoffperoxid ist.

5. System nach irgendeinem der vorhergehenden Ansprüche, wobei das System etwa 0,1 bis etwa 15 % des Oxidationsmittels aufweist.

6. System nach irgendeinem der vorhergehenden Ansprüche, wobei das System mindestens ein Färbemittel aufweist.

7. System nach irgendeinem der vorhergehenden Ansprüche, wobei die Alkalikomponenten ferner eines oder mehrere von Ammoniumcarbonat, Aminomethylpropanol, Monoisopropanolamin und Magnesiumhydroxid aufweist.

8. System nach irgendeinem der vorhergehenden Ansprüche, welches ferner eines oder mehrere Haarspülungsmittel aufweist, die in der ersten Zusammensetzung stabil sind.

9. System nach irgendeinem der vorstehenden Ansprüche, wobei die Argininverbindung ausgewählt ist aus Argininsalzen, Aminosäuren, welche eine Argininkomponente haben, und/oder Polypeptiden, welche eine Argininkomponente haben, und vorzugsweise Arginincarbonat, Argininphosphat und/oder Argininchlorid.

10. Aufhellungszusammensetzung zur Herstellung eines Systems zum Färben und/oder Aufhellen von keratinhaltigen Fasern, wobei die Aufhellungszusammensetzung aufweist:
Alkalikomponenten zum Aufhellen keratinhaltiger Fasern an einem Säugetier, wobei die Alkalikomponenten Metasilicat, Ammoniumhydroxid und Monoethanolamin aufweisen, und wobei die Zusammensetzung etwa 0,1 bis etwa 3 Gewichtsprozent des Metasilicats aufweist, wobei das Metasilicat, das Ammoniumhydroxid und das Monoethanolamin eine synergistische Aufhellung der keratinhaltigen Faser im Vergleich zu jedem des Metasilicats, Ammoniumhydroxids oder Monoethanolamins, wenn sie der Zusammensetzung allein zugesetzt werden, bewirken.

11. Nicht-therapeutisches Verfahren zur Aufhellung keratinhaltiger Faser, welches aufweist:
(a) Herstellen einer ersten Zusammensetzung, welche aufweist: (i) Alkalikomponenten zum Aufhellen keratinhaltiger Faser, wobei die Alkalikomponenten Metasilicat, Ammoniumhydroxid und Monoethanolamin aufweisen, und (ii) eine Argininverbindung,
(b) Kombinieren der ersten Zusammensetzung mit einem Oxidationsmittel, um ein System, welches einen pH-Wert von etwa 9 bis etwa 12 hat, zu bilden, und
(c) Anwenden des Systems auf keratinhaltige Faser, um die keratinhaltige Faser aufzuhellen, wobei das Metasilicat, das Ammoniumhydroxid und das Monoethanolamin eine synergistische Aufhellung der keratinhaltigen Faser im Vergleich zu jedem des Metasilicats, Ammoniumhydroxids oder Monoethanolamins, wenn sie der Zusammensetzung allein zugesetzt werden, bewirken.

12. Verfahren nach Anspruch 11, wobei das Metasilicat Natriummetasilicat ist und die mindestens zwei Alkalikomponenten ferner Ammoniumcarbonat, Aminomethylpropanol, Monoisopropanolamin und Magnesiumhydroxid aufweisen.

13. Verfahren nach Anspruch 11, wobei Schritt (a) ferner aufweist:
Herstellen einer flüssigen Zusammensetzung, welche das Metasilicat aufweist, Herstellen einer zweiten, ölphase Zusammensetzung,
Erwärmen jeder der flüssigen Zusammensetzung und der zweiten, ölphase Zusammensetzung,
Kombinieren der flüssigen Zusammensetzung und der zweiten, ölphase Zusammensetzung, um eine dritte Zusammensetzung zu bilden,
Abkühlen der dritten Zusammensetzung, und
Zugeben des Ammoniumhydroxids und des Monoethanolamins.

14. Verfahren nach Anspruch 13, wobei die flüssige Zusammensetzung und die zweite, ölphase Zusammensetzung jeweils auf etwa 75 °C bis etwa 85 °C erhitzt werden, bevor die flüssige Zusammensetzung und die zweite, ölphase Zusammensetzung kombiniert werden.

15. Verfahren nach Anspruch 13 oder 14, wobei der Schritt des Zugebens des Ammoniumhydroxids und des Monoethanolamins ferner Zugeben einer weiteren Alkalikomponente aufweist, welche von dem Metasilicat, dem Ammoniumhydroxid und dem Monoethanolamin verschieden ist.

16. Verfahren nach Anspruch 15, wobei der CIELAB dE*ab Wert mindestens etwa 1 im Vergleich zu einer Kontrolle ist.

17. Verfahren nach irgendeinem der Ansprüche 11 bis 16, wobei Schritt (c) ferner aufweist: Anwenden des Systems an einem Säugetier, welches keratinhaltige Fasern hat, und ferner Anwenden von Haarspülungsmitteln, Shampoos und/oder Haarspülungen an einem Säugetier, um Erweichen und Konditionieren einer Kopfhaut eines Säugetiers und/oder keratinhaltiger Fasern eines Säugetiers zu fördern.

## Revendications

1. Système destiné à teindre et/ou éclaircir des fibres contenant de la kératine, comportant :
(a) une première composition comportant :
(i) des composants alcalins pour éclaircir des fibres contenant de la kératine, dans lequel les composants alcalins sont présents en une quantité d'environ 0,1 à environ 10 pour-cent en poids de la première composition, dans lequel les composés alcalins comportent un métasilicate, un hydroxyde d'ammonium et une monoéthanolamine, et dans lequel la première composition comporte d'environ 0,01 à environ 3 pour-cent en poids du métasilicate, et
(ii) un composé d'arginine, dans lequel le composé d'arginine est présent dans le système en une quantité d'environ 0,1 à environ 10 pour-cent en poids pour réduire l'irritation d'une surface d'un mammifère ayant des fibres contenant de la kératine, et
(b) une composition d'agent oxydant comportant un agent oxydant, dans lequel le système a un pH d'au moins environ 9 à environ 12, dans lequel le métasilicate, l'hydroxyde d'ammonium et la monoéthanolamine produisent un éclaircissement synergique de la fibre contenant de la kératine par rapport à chaque élément parmi le métasilicate, l'hydroxyde d'ammonium et la monoéthanolamine lorsqu'ils sont ajoutés seuls à la composition.

2. Système selon la revendication 1, dans lequel le métasilicate est le métasilicate de sodium.

3. Système selon la revendication 1 ou 2, dans lequel la première composition est une solution liquide.

4. Système selon l'une quelconque des revendications précédentes, dans lequel l'agent oxydant est le peroxyde d'hydrogène.

5. Système selon l'une quelconque des revendications précédentes, dans lequel le système comporte d'environ 0,1 à environ 15 % de l'agent oxydant.

6. Système selon l'une quelconque des revendications précédentes, dans lequel le système comporte au moins un colorant.

7. Système selon l'une quelconque des revendications précédentes, dans lequel les composants alcalins comportent en outre un ou plusieurs éléments parmi du carbonate d'ammonium, de l'aminométhyl propanol, une monoisopropanol amine et de l'hydroxyde de magnésium.

8. Système selon l'une quelconque des revendications précédentes, comportant en outre un ou plusieurs agents revitalisants qui sont stables dans la première composition.

9. Système selon l'une quelconque des revendications ci-dessus, dans lequel le composé d'arginine est choisi parmi des sels d'arginine, des acides aminés ayant un composant d'arginine et/ou des polypeptides ayant un composant d'arginine, et de préférence du carbonate d'arginine, du phosphate d'arginine et/ou du chlorure d'arginine.

10. Composition d'éclaircissement pour fabriquer un système destiné à teindre et/ou éclaircir des fibres contenant de la kératine, la composition d'éclaircissement comportant :
des composants alcalins pour éclaircir des fibres contenant de la kératine sur un mammifère, dans lequel les composants alcalins comportent un métasilicate, un hydroxyde d'ammonium et une monoéthanolamine, et dans lequel la composition comporte d'environ 0,1 à environ 3 pour-cent en poids du métasilicate, dans lequel le métasilicate, l'hydroxyde d'ammonium et la monoéthanolamine produisent un éclaircissement synergique de la fibre contenant de la kératine par rapport à chaque élément parmi le métasilicate, l'hydroxyde d'ammonium et la monoéthanolamine lorsqu'ils sont ajoutés seuls à la composition.

11. Procédé non thérapeutique pour éclaircir une fibre contenant de la kératine, comportant de :
(a) préparer une première composition comportant (i) des composants alcalins pour éclaircir une fibre contenant de la kératine, dans lequel les composants alcalins comportent un métasilicate, un hydroxyde d'ammonium et une monoéthanolamine, et un composé d'arginine,
(b) combiner la première composition avec un agent oxydant pour former un système ayant un pH d'environ 9 à environ 12 et
(c) appliquer le système à une fibre contenant de la kératine pour éclaircir la fibre contenant de la kératine, dans lequel le métasilicate, l'hydroxyde d'ammonium et la monoéthanolamine produisent un éclaircissement synergique de la fibre contenant de la kératine par rapport à chaque élément parmi le métasilicate, l'hydroxyde d'ammonium et la monoéthanolamine lorsqu'ils sont ajoutés seuls à la composition.

12. Procédé selon la revendication 11, dans lequel le métasilicate est un métasilicate de sodium et les au moins deux composants alcalins comportent en outre du carbonate d'ammonium, du propanol d'aminométhyle, de l'amine de monoisopropanol et de l'hydroxyde de magnésium.

13. Procédé selon la revendication 11, dans lequel l'étape (a) comporte en outre de :
préparer une composition liquide comportant le métasilicate,
préparer une deuxième composition en phase huileuse,
chauffer chaque composition parmi la composition liquide et la deuxième composition en phase huileuse,
combiner la composition liquide et la deuxième composition en phase huileuse pour former une troisième composition,
refroidir la troisième composition, et
ajouter l'hydroxyde d'ammonium et la monoéthanolamine.

14. Procédé selon la revendication 13, dans lequel la composition liquide et la deuxième composition en phase huileuse sont chacune chauffées jusqu'à environ 75 °C à environ 85 °C avant de combiner la composition liquide et la deuxième composition en phase huileuse.

15. Procédé selon la revendication 13 ou 14, dans lequel l'étape d'ajout de l'hydroxyde d'ammonium et de la monoéthanolamine comporte en outre d'ajouter un composant alcalin supplémentaire qui est différent du métasilicate, de l'hydroxyde d'ammonium et de la monoéthanolamine.

16. Procédé selon la revendication 15, dans lequel la valeur CIELAB dE*ab est au moins d'environ 1 par rapport à un témoin.

17. Procédé selon l'une quelconque des revendications 11 à 16, dans lequel l'étape (c) comporte en outre d'appliquer le système à un mammifère ayant des fibres contenant de la kératine et d'appliquer également des agents revitalisants, des shampooings et/ou des après-shampoings à un mammifère pour favoriser l'assouplissement et la revitalisation d'un cuir chevelu d'un mammifère et/ou de fibres contenant de la kératine d'un mammifère.
